# FASCICULE DE BREVET EUROPEEN

(11) **EP 1 174 113 B1**
(45) Date de publication et mention de la délivrance du brevet: **07.09.2005**
(21) Numéro de dépôt: 01401837.8
(22) Date de dépôt: 10.07.2001
(51) Int. Cl.: A61K 7/06, A61K 7/00

(54) **Composition de coiffage des cheveux permettant un remodelage de la coiffure et procédé de remodelage de la coiffure utilisant une telle composition**
Haarformungszusammensetzung zur Haarfestigung und Verfahren zur Haarfestigung mit dieser Zusammensetzung
Hairstyling composition capable of remodelling the hair and process of remodelling the hair with said composition

(30) Priorité: 18.07.2000 FR 0009406
(43) Date de publication de la demande: 23.01.2002
(73) Titulaire: L'OREAL, 75008 Paris (FR)
(72) Inventeur: Giroud, Franck, F-92110 Clichy (FR); Samain, Henri, F-91570 Bièvres (FR)
(74) Mandataire: Casalonga, Axel

(56) Documents cités:
- EP-A- 0 761 199
- EP-A- 0 919 219
- EP-A- 1 004 288
- WO-A-98/25710
- FR-A- 2 749 568
- US-A- 4 172 122
- US-A- 5 593 680

## Description

La présente invention concerne une composition de coiffage des cheveux permettant un remodelage de la coiffure, ainsi qu'un procédé de remodelage de la coiffure utilisant une telle composition.

Il existe de nombreux produits de coiffage sous forme de laques, de sprays, de gels ou de lotions. Ces produits sont des solutions ou dispersions de polymères qui, après évaporation de la phase solvant, assurent la fixation et le maintien de la coiffure grâce à la formation de films polymériques entourant chaque cheveu à la manière d'une gaine et grâce à l'établissement de liens physiques entre les cheveux.

Ce type de fixation de la coiffure est définitif puisque toute modification importante de la forme de la coiffure entraîne la rupture irréversible des jonctions et altère par conséquent le maintien de la coiffure. Les produits de coiffage usuels ne permettent donc pas un remodelage de la coiffure sans apport de produit coiffant supplémentaire.

La demanderesse s'est aperçu qu'il était possible de surmonter cet inconvénient des produits coiffants connus en associant aux polymères fixants utilisés communément en coiffure, un polymère thermofusible cristallin, à bas point de fusion cristalline.

L'adjonction de ce polymère thermofusible aux produits fixants usuels se traduit par une "plastification" du film polymérique entourant les cheveux aux températures proches ou supérieures à la température de fusion cristalline dudit polymère thermofusible cristallin.

Les cheveux ainsi entourés d'une gaine devenue plastique par chauffage peuvent de nouveau être coiffés et mis en forme. Le simple refroidissement de la coiffure à une température inférieure à la température de fusion cristalline du polymère thermofusible se traduira par une resolidification de la composition fixante, par l'établissement de nouvelles jonctions physiques entre les cheveux et donc par un maintien équivalent à celui obtenu lors de la première pulvérisation du produit fixant

La "thermoplasticité" de la composition à des températures relativement basses permet ainsi un remodelage de la coiffure sans apport de matière coiffante supplémentaire.

La présente invention a par conséquent pour objet une composition de coiffage contenant, dans un milieu liquide physiologiquement acceptable, l'association d'au moins un polymère fixant filmogène, et d'au moins un polymère thermofusible cristallin, soluble dans ledit milieu, ayant une température de fusion cristalline, mesurée par analyse enthalpique différentielle, comprise entre 30 °C et 80 °C et constitué
i) de 85 à 98 % en poids de motifs hydrophobes dérivés de monomères α,β-éthyléniques à chaîne latérale n-alkyle en C₁₂₋₅₀, de préférence en C₁₄₋₂₄, formant des homopolymères cristallins et
ii) de 2 à 15 % en poids de motifs hydrophiles dérivés d'acides monocarboxyliques en C₃₋₆ α,β-insaturés, d'acides dicarboxyliques insaturés en C₄₋₆, des esters et amides à chaîne alkyle en C₁₋₄ de ces acides monocarboxyliques ou dicarboxyliques, du méthacrylate d'hydroxyéthyle et de la vinylpyrrolidone.

Elle a en outre pour objet un procédé de modelage ou de remodelage des cheveux consistant à appliquer sur la chevelure sèche ou humide la composition de coiffage ci-dessus, à donner à la chevelure la forme souhaitée en chauffant les cheveux au moyen d'une source de chaleur appropriée jusqu'à une température proche ou supérieure à la température de fusion cristalline du polymère thermofusible cristallin contenu dans la composition de coiffage, et à laisser refroidir la chevelure jusqu'à une température inférieure à ladite température de fusion cristalline dudit polymère thermofusible cristallin, ces deux dernières étapes pouvant être répétées un grand nombre de fois sans apport de composition de coiffage supplémentaire.

On peut utiliser pour la présente invention tout polymère fixant filmogène connu en tant que tel dans le domaine des traitements capillaires, ainsi que bien entendu également des mélanges contenant plusieurs de ces polymères. On distingue classiquement les polymères fixants cationiques, anioniques, amphotères et non ioniques.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5 000 000 et de préférence entre 1000 et 3 000 000.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs suivants : 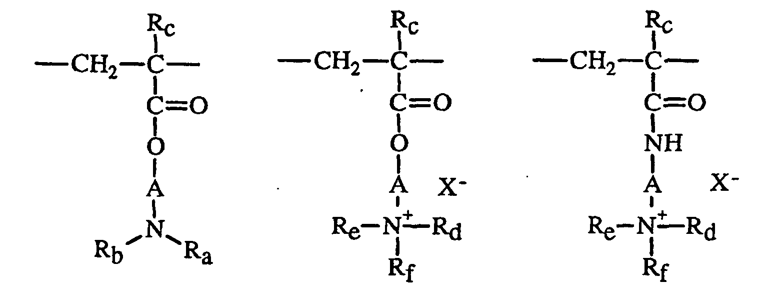 dans lesquelles
   Rₐ et R_{b} représentent chacun un atome d'hydrogène ou un groupe alkyle en C₁₋₆,
   R_{c} désigne un atome d'hydrogène ou un radical CH₃,
   R_{d}, Rₑ et R_{f}, identiques ou différents, représentent chacun un groupe alkyle en C₁₋₁₈ ou un radical benzyle,
   A est un groupe alkyle linéaire ou ramifié en C₁₋₆ ou un groupe hydroxyalkyle en C₁₋₄, et
   X⁻ désigne un anion méthosulfate ou halogénure tel qu'un ion chlorure ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieurs, des acides acryliques ou méthacryliques ou leurs esters, des vinyllactames tels que la vinylpyrrolidone ou le vinylcaprolactame, et des esters vinyliques.
   Ainsi, on peut citer parmi les copolymères de la famille (1)
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT® P 100 par la société CIBA GEIGY,
   - le copolymère d'acrylamide et de méthosulfate de méthacryloyloxyéthyltriméthylammonium vendu sous la dénomination RETEN® par la société HERCULES,
   - les copolymères vinylpyrrolidone/acrylate ou méthacrylate de dialkylaminoalkyle quaternisés ou non, tels que les produits vendus sous la dénomination GAFQUAT® par la société ISP, comme par exemple GAFQUAT® 734 ou GAFQUAT® 755, ou bien les produits dénommés COPOLYMER® 845, 958 et 937. Ces polymères sont décrits en détail dans les brevets français 2.077.143 et 2.393.573,
   - les terpolymères méthacrylate de diméthylaminoéthyle/vinylcaprolactame/vinylpyrrolidone tels que le produit commercialisé sous la dénomination GAFFIX VC 713 par la société ISP, et
   - le copolymère vinylpyrrolidone/méthacrylamide de diméthylaminopropyle quaternisé tel que le produit vendu sous la dénomination GAFQUAT® HS 100 par la société ISP.
(2) les polysaccharides quaternisés décrits plus particulièrement dans les brevets américains 3.589.578 et 4.031.307 tels que les gommes de guar contenant des groupements cationiques trialkylammonium.
   De tels produits sont commercialisés notamment sous les dénominations commerciales de JAGUAR® C13 S, JAGUAR® C 15 et JAGUAR® C 17 par la société MEYHALL.
(3) les copolymères quaternaires de vinylpyrrolidone et de vinylimidazole tels que les produits commercialisés par BASF sous la dénomination LUVIQUAT® TFC,
(4) les chitosanes ou leurs sels, en particulier les acétate, lactate, glutamate, gluconate ou pyrrolidonecarboxylate de chitosane.
   On peut citer le chitosane ayant un taux de désacétylation de 90,5 % en poids vendu sous la dénomination KYTAN BRUT STANDARD® par la société ABER TECHNOLOGIES, le pyrrolidonecarboxylate de chitosane vendu sous la dénomination KYTAMER® PC par la société AMERCHOL.
(5) les dérivés de cellulose cationiques tels que les copolymères de cellulose ou les dérivés de cellulose greffés avec un monomère hydrosoluble comportant un groupe ammonium quaternaire et décrits notamment dans le brevet US 4 131 576 tels que les hydroxyalkylcelluloses, comme les hydroxyméthylhydroxyéthyl- ou hydroxypropylcelluloses greffées notamment avec un sel de méthacryloyloxyéthyltriméthylammonium, de méthacrylamidopropyltriméthylammonium ou de diméthyldiallylammonium.

Les produits commercialisés répondant à cette définition sont plus particulièrement les produits vendus sous la dénomination CELQUAT® L 200 et CELQUAT® H 100 par la société NATIONAL STARCH.

Les polymères fixants anioniques généralement utilisés sont des polymères comportant des groupements dérivés d'un acide carboxylique, sulfonique ou phosphorique et ont un poids moléculaire moyen en poids compris entre environ 500 et 5 000 000.

Les groupements acide carboxylique sont apportés par des monomères insaturés contenant une ou deux fonctions acide carboxylique, tels que ceux répondant à la formule: 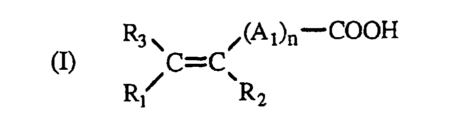 dans laquelle n est un nombre entier de 0 à 10, A₁ désigne un groupement méthylène, éventuellement relié à l'atome de carbone du groupement insaturé ou du groupement méthylène voisin, lorsque n est supérieur à 1, par l'intermédiaire d'un hétéroatome tel que oxygène ou soufre, R₃ désigne un atome d'hydrogène, un groupement phényle ou benzyle, R₁ désigne un atome d'hydrogène, un groupement alkyle inférieur ou carboxyle, et R₂ désigne un atome d'hydrogène, un groupement alkyle inférieur, un groupement -CH₂-COOH, phényle ou benzyle.

Dans la formule précitée un radical alkyle inférieur désigne de préférence un groupement ayant de 1 à 4 atomes de carbone et en particulier un groupe méthyle ou éthyle.

Les polymères fixants anioniques carboxylés préférés selon l'invention sont:
A) les homo- ou copolymères d'acide acrylique ou méthacrylique ou leurs sels et en particulier les produits commercialisés sous les dénominations VERSICOL® E ou K par la société ALLIED COL-LOID, et sous la dénomination ULTRAHOLD® par la société BASF ; les copolymères d'acide acrylique et d'acrylamide commercialisés sous forme de sel de sodium sous les dénominations RETEN® 421, 423 ou 425 par la Société HERCULES ; les sels de sodium des acides polyhydroxycarboxyliques.
B) les copolymères d'acide acrylique ou d'acide méthacrylique et d'un monomère monoéthylénique tel que l'éthylène, le styrène, les esters vinyliques, les esters d'acide acrylique ou méthacrylique.
   Ces copolymères peuvent être greffés sur un polyalkylène glycol tel que le polyéthylèneglycol et sont éventuellement réticulés.
   De tels polymères sont décrits en particulier dans le brevet français FR 1 222 944 et dans la demande de brevet allemand DE 2 330 956. On peut notamment citer les copolymères comportant dans leur chaîne un motif acrylamide éventuellement N-alkylé et/ou hydroxyalkylé, tels que ceux décrits dans les demandes de brevet luxembourgeois LU 75370 et LU 75371 ou proposés sous la dénomination QUADRAMER® par la Société AMERICAN CYANAMID.
   On peut également citer les copolymères d'acide acrylique et de méthacrylate d'alkyle en C₁-C₄, et les terpolymères de vinylpyrrolidone, d'acide (méth)acrylique et de (méth)acrylate d'alkyle en C₁-C₂₀, par exemple de lauryle (ACRYLIDONE® LM de la société ISP), de tertiobutyle (LUVIFLEX® VBM 70 commercialisé par BASF) ou de méthyle (STEPANHOLD® EXTRA commercialisé par STEPAN), et les terpolymères acide méthacrylique/acrylate d'éthyle/acrylate de tertiobutyle tel que le produit commercialisé sous la dénomination LUVIMER® 100 P par la société BASF.
C) les copolymères dérivés d'acide crotonique tels que ceux comportant dans leur chaîne des motifs acétate ou propionate de vinyle et éventuellement d'autres monomères tels que les esters allylique, méthallylique ou vinylique d'un acide carboxylique saturé, linéaire ou ramifié, à longue chaîne hydrocarbonée comportant au moins 5 atomes de carbone, ces polymères pouvant éventuellement être greffés et réticulés, ou encore les esters vinylique, allylique ou méthallylique d'un acide carboxylique α- ou β-cyclique.
   De tels polymères sont décrits, entre autres, dans les brevets français FR 1 222 944, FR 1 580 545, FR 2 265 782, FR 2 265 781, FR 1 564 110 et FR 2 439 798.
   On peut citer à titre d'exemples de produits commerciaux entrant dans cette classe les résines 28-29-30, 26-13-14 et 28-13-10 commercialisées par la société NATIONAL STARCH.
D) les copolymères dérivés d'acides ou d'anhydrides carboxyliques monoinsaturés en C₄-C₈ choisis parmi:
   - les copolymères comprenant
      (i) un ou plusieurs acides ou anhydrides maléique, fumarique, itaconique et
      (ii) au moins un monomère choisi parmi les esters vinyliques, les éthers vinyliques, les halogénures vinyliques, les dérivés phénylvinyliques, l'acide acrylique et ses esters, les fonctions anhydride de ces copolymères étant éventuellement monoestérifiées ou monoamidifiées;

      De tels polymères sont décrits en particulier dans les brevets US 2,047,398, US 2,723,248, US 2,102,113 et GB 839,805 et notamment ceux commercialisés sous les dénominations GANTREZ AN ou ES, AVANTAGE® CP par la société ISP;
      - les copolymères comprenant (i) un ou plusieurs anhydrides maléique, citraconique ou itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

      Ces polymères sont par exemple décrits dans les brevets français FR 2 350 384 et FR 2 357 241 de la demanderesse.
E) les polyacrylamides comportant des groupements carboxylate.

Les groupements anioniques des polymères fixants anioniques de la présente invention peuvent également être des groupes acide sulfonique apportés par des motifs vinylsulfonique, styrènesulfonique, naphtalènesulfonique ou acrylamidoalkylsulfonique.

Ces polymères à groupes acide sulfonique sont notamment choisis parmi:
- les sels de poly(acide vinylsulfonique) ayant un poids moléculaire moyen en poids compris entre environ 1000 et 100 000 ainsi que les copolymères d'acide vinylsulfonique et d'un comonomère insaturé tel que l'acide acrylique, l'acide méthacrylique, les esters de ces acides, l'acrylamide, les dérivés d'acrylamide, les éthers vinyliques et la vinylpyrrolidone ;
- les sels de poly(acide styrènesulfonique). On peut citer à titre d'exemple deux sels de sodium ayant un poids moléculaire moyen en poids d'environ 500 000 et d'environ 100 000 commercialisés respectivement sous les dénominations FLEXAN® 500 et FLEXAN® 130 par la société NATIONAL STARCH. Ces composés sont décrits dans le brevet FR 2 198 719 ;
- les sels de poly(acide acrylamidosulfonique), tels que ceux mentionnés dans le brevet US 4,128,631 et plus particulièrement le poly(acide acrylamidoéthylpropanesulfonique) commercialisé sous la dénomination COSMEDIA POLYMER® HSP 1180 par la société HEN-KEL.

Selon l'invention, les polymères fixants anioniques sont de préférence choisis parmi les copolymères d'acide acrylique tels que les terpolymères acide acrylique/acrylate d'éthyle/N-tertiobutylacrylamide commercialisés notamment sous la dénomination ULTRAHOLD STRONG® par la société BASF, les copolymères dérivés d'acide crotonique tels que les terpolymères acétate de vinyle/tertio-butyl benzoate de vinyle/acide crotonique et les terpolymères acide crotonique/acétate de vinyle/néododécanoate de vinyle commercialisés notamment sous la dénomination Résine 28-29-30 par la société NATIONAL STARCH, les copolymères dérivés d'acide ou d'anhydride maléique, fumarique ou itaconique et contenant comme comonomères des esters vinyliques, des éthers vinyliques, des halogénures vinyliques, des dérivés phénylvinyliques, l'acide acrylique et les esters d'acide acrylique, tels que les copolymères méthylvinyléther/anhydride maléique monoestérifié, commercialisés par exemple sous la dénomination GANTREZ® par la société ISP, les copolymères d'acide méthacrylique et de méthacrylate de méthyle commercialisés sous la dénomination EUDRAGIT® L par la société ROHM PHARMA, les copolymères acide méthacrylique/méthacrylate de méthyle/acrylate d'alkyle en C₁₋₄ /acide acrylique ou méthacrylate d'hydroxyalkyle en C₁₋₄, commercialisés sous la dénomination AMERHOLD® DR 25 par la société AMERCHOL, ou sous la dénomination ACUDYNE® 255 par la société ROHM & HAAS, les copolymères d'acide méthacrylique et d'acrylate d'éthyle commercialisés sous la dénomination LUVIMER® MAEX ou MAE par la société B ASF et les copolymères acétate de vinyle/acide crotonique et les copolymères acétate de vinyle/acide crotonique greffés par du polyéthylèneglycol commercialisés sous la dénomination ARISTOFLEX® A par la société BASF.

Les polymères fixants amphotères utilisables pour la présente l'invention sont choisis notamment parmi les polymères comportant des motifs B et C répartis statistiquement dans la chaîne polymère, où B désigne un motif dérivant d'un monomère comportant au moins un atome d'azote basique et C désigne un motif dérivant d'un monomère comportant un ou plusieurs groupements acide carboxylique ou acide sulfonique. Les polymères fixants amphotères peuvent également comporter des motifs zwittérioniques de type carboxybétaïne ou sulfobétaïne. Il peut également s'agir de polymères à chaîne principale cationique comportant des groupements amine primaire, secondaire, tertiaire ou quaternaire, parmi lesquels au moins un porte, par l'intermédiaire d'un radical hydrocarboné, un groupement acide carboxylique ou acide sulfonique. Les polymères fixant amphotères peuvent encore avoir une chaîne anionique dérivée d'acides dicarboxyliques α,β-insaturés dont l'un des groupements carboxyle a été amené à réagir avec une polyamine comportant un ou plusieurs groupements amine primaire ou secondaire.

Les polymères fixants amphotères répondant à la définition donnée ci-dessus sont choisis en particulier parmi les polymères suivants:
(1) Les polymères résultant de la copolymérisation d'un monomère vinylique portant un groupement acide carboxylique tel que l'acide acrylique, l'acide méthacrylique, l'acide maléique, l'acide α-chloroacrylique, et d'un monomère vinylique contenant au moins une fonction basique tel que les méthacrylate et acrylate de dialkylaminoalkyle ou les dialkylaminoalkyl(méth)acrylamides. De tels composés sont décrits par exemple dans le brevet américain US 3,836,537.
(2) Les polymères comportant des motifs dérivés :
   (a) d'au moins un monomère choisi parmi les acrylamides ou méthacrylamides N-alkylés,
   (b) d'au moins un comonomère contenant une ou plusieurs fonctions acide carboxylique, et
   (c) d'au moins un comonomère basique tel que des esters à substituants amine primaire, secondaire, tertiaire ou quaternaire d'acide acrylique et d'acide méthacrylique, et le produit de quaternisation du méthacrylate de diméthylaminoéthyle avec le sulfate de diméthyle ou diéthyle.

   Les acrylamides ou méthacrylamides N-alkylés (a) préférés sont ceux portant des radicaux alkyle en C₂₋₁₂, tels que le N-éthylacrylamide, le N-tertiobutylacrylamide, le N-tertio-octylacrylamide, le N-octylacrylamide, le N-décylacrylamide, le N-dodécylacrylamide ainsi que les méthacrylamides correspondants.
   Les comonomères à groupe acide carboxylique (b) sont choisis plus particulièrement parmi les acides acrylique, méthacrylique, crotonique, itaconique, maléique, fumarique ainsi que parmi les monoesters d'alkyle en C₁₋₄ des acides ou des anhydrides maléique ou fumarique.
   Les comonomères basiques (c) préférés sont le méthacrylate d'aminoéthyle, le méthacrylate de butylaminoéthyle, le méthacrylate de N,N'-diméthylaminoéthyle et le méthacrylate de N-tertiobutylaminoéthyle.
   On utilise en particulier les copolymères dont la dénomination CTFA(4^{ème} Ed., 1991) est "Octylacrylamide/acrylates/butylaminoethylmethacrylate copolymer", tels que les produits commercialisés sous la dénomination AMPHOMER® ou LOVOCRYL® 47 par la société NATIONAL STARCH.
(3) Les polyaminoamides réticulés et alcoylés, dérivés en partie ou en totalité de polyaminoamides de formule générale:

   (II) -[C(=O)-R₄-C(=O)-Z-]-

   dans laquelle R₄ représente un radical divalent dérivé d'un acide dicarboxylique saturé, d'un acide aliphatique mono- ou dicarboxylique à double liaison éthylénique, d'un ester d'alkyle en C₁₋₆ de ces acides ou d'un radical dérivant de l'addition de l'un quelconque desdits acides sur une amine bis primaire ou bis secondaire, et Z désigne un radical d'une polyalkylène-polyamine bis-primaire, mono- ou bis-secondaire, et de préférence représente:
   a) dans les proportions de 60 à 100 % en moles, le radical

      (III) -NH-[(CH₂)ₓ-NH]ₚ-

      où x = 2 et p = 2 ou 3, ou bien x = 3 et p = 2
      ce radical dérivant de la diéthylènetriamine, de la triéthylènetétraamine ou de la dipropylènetriamine;
   b) dans les proportions de 0 à 40 % en moles le radical de formule (III) dans lequel x = 2 et p = 1, dérivé de l'éthylènediamine, ou le radical 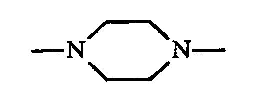 dérivé de la pipérazine:
   c) dans les proportions de 0 à 20 % en moles le radical -NH-(CH₂)₆-NH- dérivé de l'hexaméthylènediamine,
      ces polyaminoamines étant réticulées par addition de 0,025 à 0,35 mole par mole de groupement amine, d'un agent réticulant bifonctionnel choisi parmi les épihalohydrines, les diépoxydes, les dianhydrides, les composés di-insaturés, et alcoylés par l'acide acrylique, l'acide chloracétique ou une alcane-sultone.

   Les acides carboxyliques saturés sont choisis de préférence parmi les acides ayant 6 à 10 atomes de carbone tels que l'acide adipique, l'acide triméthyl-2,2,4-adipique et triméthyl-2,4,4-adipique, l'acide téréphtalique, les acides à double liaison éthylénique comme par exemple les acides acrylique, méthacrylique, itaconique.
   Les alcane-sultones utilisées dans l'alcoylation sont de préférence la propanesultone ou la butanesultone.
   Les sels des agents d'alcoylation sont de préférence les sels de sodium ou de potassium.
(4) Les polymères comportant des motifs zwittérioniques de formule: 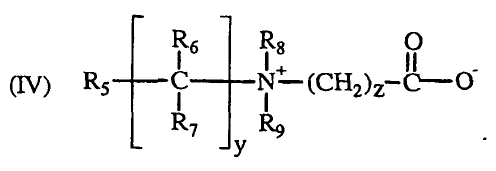 dans laquelle R₅ désigne un groupement insaturé polymérisable tel qu'un groupement acrylate, méthacrylate, acrylamide ou méthacrylamide, y et z représentent chacun un nombre entier de 1 à 3, R₆ et R₇ représentent chacun indépendamment un atome d'hydrogène ou un groupe méthyle, éthyle ou propyle, R₈ et R₉ représentent chacun indépendamment un atome d'hydrogène ou un radical alkyle, le nombre total d'atomes de carbone dans R₈ et R₉ ne dépassant pas 10.
   Les polymères comprenant de tels motifs de formule (IV) peuvent comporter en outre des motifs dérivés de monomères non zwittérioniques tels que l'acrylate ou le méthacrylate de diméthyle ou de diéthylaminoéthyle, les acrylates ou méthacrylates d'alkyle, les acrylamides ou méthacrylamides ou l'acétate de vinyle.
   A titre d'exemple, on peut citer le copolymère méthacrylate de méthyle/méthacrylate de diméthylcarboxyméthylammonioéthyle tel que le produit commercialisé sous la dénomination DIAFORMER® Z301 par la société SANDOZ.
(5) Les polymères dérivés du chitosane comportant des motifs monomères répondant aux formules suivantes: 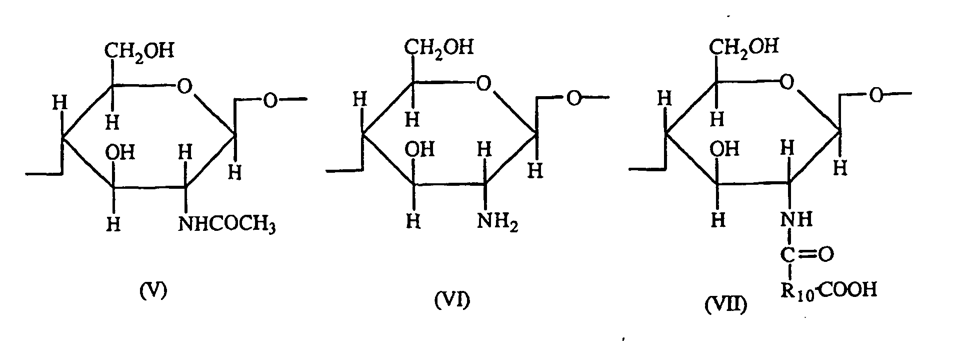 le motif de formule (V) étant présent dans des proportions comprises entre 0 et 30 %, le motif de formule (VI) dans des proportions comprises entre 5 et 50 % et le motif de formule (VII) dans des proportions comprises entre 30 et 90 %, étant entendu que dans ce motif (VII), R₁₀ représente un radical de formule: 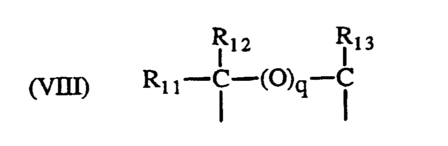 dans laquelle
   si q = 0, alors R₁₁, R₁₂ et R₁₃, identiques ou différents, représentent chacun un atome d'hydrogène, un groupe méthyle, hydroxyle, acétoxy ou amino, un groupe monoalcoylamine ou dialcoylamine éventuellement interrompu par un ou plusieurs atomes d'azote et/ou éventuellement substitués par un ou plusieurs groupes amine, hydroxyle, carboxyle, alcoylthio ou sulfo, un groupe alcoylthio dont le groupe alcoyle porte un reste amino, l'un au moins des radicaux R₁₁, R₁₂ et R₁₃ étant dans ce cas un atome d'hydrogène; ou
   si q = 1, alors R₁₁, R₁₂ et R₁₃ représentent chacun un atome d'hydrogène,
   ainsi que les sels formés par ces composés avec des bases ou des acides.
(6) Les polymères obtenus par N-carboxyalkylation du chitosane, comme le N-carboxyméthylchitosane ou le N-carboxybutylchitosane commercialisé sous la dénomination EVALSAN® par la société JAN DEKKER.
(7) Les polymères répondant à la formule générale (IX) 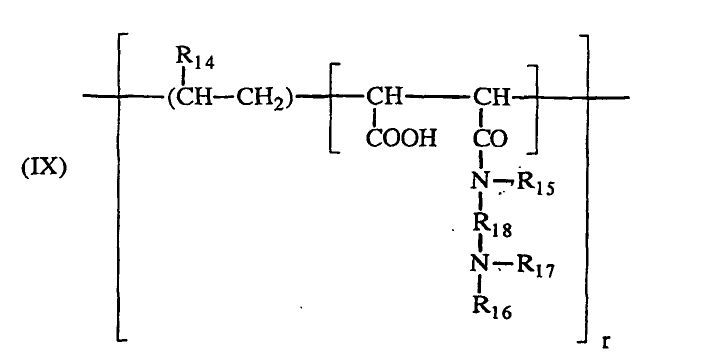 décrits notamment dans le brevet FR 1 400 366, dans laquelle R₁₄ représente un atome d'hydrogène ou un radical CH₃O, CH₃CH₂O ou phényle, R₁₅ désigne un atome d'hydrogène ou un radical alkyle inférieur tel que méthyle ou éthyle, R₁₆ désigne un atome d'hydrogène ou un radical alkyle inférieur tel que méthyle ou éthyle, R₁₇ désigne un radical alkyle inférieur tel que méthyle ou éthyle ou un radical répondant à la formule:

   -R₁₈-N(R₁₆)₂,

   R₁₈ représentant un groupement -CH₂-CH₂-, -CH₂-CH₂-CH₂-, -CH₂-CH(CH₃)-, et R₁₆ ayant les significations mentionnées ci-dessus, ainsi que les homologues supérieurs de ces radicaux contenant jusqu'à 6 atomes de carbone.
(8) Les polymères amphotères du type -D-X-D-X- choisis parmi:
   (a) les polymères obtenus par action d'acide chloroacétique ou de chloroacétate de sodium sur les composés comportant au moins un motif de formule:

      (X) -D-X-D-X-D-

      où D désigne un radical 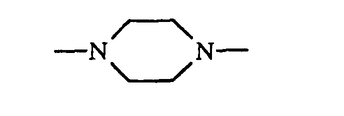 et X désigne le symbole E ou E', E ou E' identiques ou différents désignant un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote ou de soufre, 1 à 3 cycles aromatiques et/ou hétérocycliques, les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne,
   (b) les polymères de formule:

      -D-X'-D-X'- (X')

      où D désigne un radical 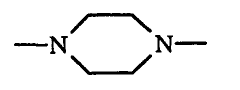 et X' désigne le symbole E ou E' et au moins une fois E', E ayant la signification indiquée ci-dessus et E' étant un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloroacétique ou du chloroacétate de soude.
(9) Les copolymères alkyl(C₁₋₅)vinyléther/anhydride maléique modifiés partiellement par semi-amidification avec une N,N-dialkylaminoalkylamine telle que la N,N-diméthylaminopropylamine, ou par semiestérification avec une N,N-dialcanolamine. Ces copolymères peuvent également comporter d'autres comonomères vinyliques tels que le vinylcaprolactame.

Les polymères fixants amphotères préférés selon l'invention sont ceux de la famille (3) décrite ci-dessus, tels que ceux dont la dénomination CTFA est "Octylacrylamide/acrylates/butylaminoethylmethacrylate copolymer". On peut citer à titre d'exemple les produits commercialisés sous les dénominations AMPHOMER®, AMPHOMER® LV 71 ou LOVOCRYL® 47 par la société NATIONAL STARCH.

D'autres polymères fixants amphotères préférés sont ceux de la famille (4), comme par exemple les copolymères de méthacrylate de méthyle et de méthacrylate de diméthylcarboxyméthylammonioéthyle, commercialisés par exemple sous la dénomination DIAFORMER® Z301 par la société SANDOZ.

Les polymères fixants anioniques ou amphotères peuvent, si nécessaire, être partiellement ou totalement neutralisés. Les agents de neutralisation sont par exemple la soude, la potasse, l'amino-2-méthylpropanol, la monoéthanolamine, la triéthanolamine ou la tri-isopropanolamine, les acides minéraux ou organiques tels que l'acide chlorhydrique ou l'acide citrique.

Les polymères fixants non ioniques utilisables selon la présente invention sont choisis par exemple parmi :
- les homopolymères de vinylpyrrolidone,
- les copolymères de vinylpyrrolidone et d'acétate de vinyle,
- les polyalkyloxazolines telles que les polyéthyloxazolines proposées par la société DOW CHEMICAL sous les dénominations PEOX® 50 000, PEOX® 200 000 et PEOX® 500 000,
- les homopolymères d'acétate de vinyle tels que le produit proposé sous le nom de APPRETAN® EM par la société HOECHST ou le produit proposé sous le nom de RHODOPAS® A 012 par la société RHONE POULENC,
- les copolymères d'acétate de vinyle et d'esters acryliques tels que le produit proposé sous la dénomination RHODOPAS® AD 310 par RHONE POULENC,
- les copolymères d'acétate de vinyle et d'éthylène tels que le produit proposé sous le nom de APPRETAN® TV par la société HOECHST,
- les copolymères d'acétate de vinyle et d'ester maléique par exemple de maléate de dibutyle tels que le produit proposé sous le nom de APPRETAN® MB EXTRA par la société HOECHST,
- les copolymères d'éthylène et d'anhydride maléique,
- les homopolymères d'acrylates d'alkyle et les homopolymères de méthacrylates d'alkyle tels que le produit proposé sous la dénomination MICROPEARL® RQ 750 par la société MATSUMOTO ou le produit proposé sous la dénomination LUHYDRAN® A 848 S par la société BASF,
- les copolymères d'esters acryliques tels que par exemple les copolymères d'acrylates d'alkyle et de méthacrylates d'alkyle tels que les produits proposés par la société ROHM & HAAS sous les dénominations PRIMAL AC-261 K et EUDRAGIT NE 30 D, par la société BASF sous les dénominations ACRONAL® 601, LUHYDRAN® LR 8833 ou 8845, et par la société HOECHST sous les dénominations APPRETAN® N 9213 ou N9212,
- les copolymères d'acrylonitrile et d'un monomère non ionique choisi par exemple parmi le butadiène et les (méth)acrylates d'alkyle ; on peut citer les produits proposés sous les dénominations NIPOL® LX 531 B par la société NIPPON ZEON ou ceux proposés sous la dénomination CJ 0610 B par la société ROHM & HAAS,
- les polyuréthannes tels que les produits proposés sous les dénominations ACRYSOL® RM 1020 ou ACRYSOL® RM 2020 par la société ROHM & HAAS, les produits URAFLEX® XP 401 UZ, URAFLEX® XP et 402 UZ par la société DSM RESINS,
- les copolymères d'acrylate d'alkyle et d'uréthanne tels que le produit 8538-33 commercialisé par le société NATIONAL STARCH,
- les polyamides tels que le produit ESTAPOR® LO 11 proposé par la société RHONE POULENC,
- les gommes de guar non ioniques chimiquement modifiées ou non modifiées.

Les gommes de guar non ioniques non modifiées sont par exemple les produits vendus sous la dénomination VIDOGUM® GH 175 par la société UNIPECTINE et sous la dénomination JAGUAR® C par la société MEYHALL. Les gommes de guar non ioniques modifiées utilisables selon l'invention sont de préférence modifiées par des groupements hydroxyalkyle en C₁₋₆. On peut mentionner à titre d'exemple les groupements hydroxyméthyle, hydroxyéthyle, hydroxypropyle et hydroxybutyle.

Ces gommes de guar sont bien connues dans la technique et peuvent par exemple être préparées par réaction des oxydes d'alcènes correspondants, tels que par exemple des oxydes de propylène, avec la gomme de guar de façon à obtenir une gomme de guar modifiée par des groupements hydroxypropyle.

De telles gommes de guar non ioniques éventuellement modifiées par des groupements hydroxyalkyle sont par exemple vendues sous les dénominations commerciales JAGUAR® HP8, JAGUAR® HP60 et JAGUAR®HP120, JAGUAR®DC293 et JAGUAR® HP 105 par la société MEYHALL, ou sous la dénomination GALACTASOL® 4H4FD2 par la société AQUALON.

Selon l'invention, on peut également utiliser, en tant que polymères fixants, des polymères filmogènes de type silicone greffée comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4 693 935, US 4 728 571 et US 4 972 037.

Ces polymères sont de préférence anioniques ou non ioniques.

De tels polymères sont par exemple les copolymères susceptibles d'être obtenus par polymérisation radicalaire à partir du mélange de monomères formé
a) de 50 à 90 % en poids d'acrylate de tertiobutyle,
b) de 0 à 40 % en poids d'acide acrylique,
c) de 5 à 40 % en poids d'un macromère siliconé de formule 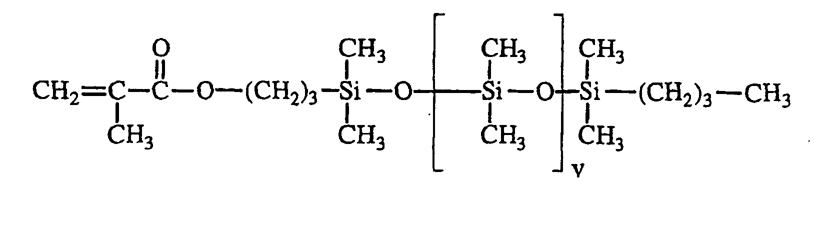 avec v étant un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser comme polymères fixants filmogènes des polyuréthannes fonctionnalisés ou non, siliconés ou non.

Les polyuréthannes particulièrement visés par la présente invention sont ceux décrits dans les brevets EP 0 751 162, EP 0 637 600, FR 2 743 297 et EP 0 648 485 de la demanderesse, ainsi que le brevet EP 0 656 021 ou WO 94/03510 de la société BASF et EP 0 619 111 de la société NATIONAL STARCH.

Les polymères fixants filmogènes décrits ci-dessus ont, certes, un pouvoir fixant satisfaisant permettant un bon maintien de la coiffure au cours du temps, mais ils ne présentent pas un caractère thermoplastique suffisant pour permettre un remodelage thermique de la coiffure. Comme indiqué dans l'introduction, seule l'utilisation *conjointe* d'un ou de plusieurs polymères fixants classiques et d'au moins un polymère thermofusible cristallin décrit plus en détail ci-après permet l'obtention de compositions coiffantes alliant un bon pouvoir fixant et d'excellentes propriétés thermoplastiques.

La quantité de polymère thermofusible cristallin nécessaire pour obtenir l'effet recherché, c'est-à-dire une thermoplasticité suffisante pour permettre un remodelage à chaud de la coiffure, dépend entre autres de la nature chimique des deux types de polymères, de la masse molaire de ces polymères et du pouvoir fixant recherché et peut varier entre de larges limites.

De manière générale, le rapport en poids de la quantité totale de polymère cristallin thermofusible à la quantité totale de polymère fixant filmogène est compris entre 1/10 000 et 3/1, et de préférence entre 1/200 et 3/5.

Les polymères thermofusibles cristallins utilisables selon la présente invention sont des copolymères cristallins comprenant
i) de 85 à 98 % en poids de motifs hydrophobes et
ii) de 2 à 15 % en poids de motifs hydrophiles.

Les motifs hydrophobes sont dérivés de monomères α,β-éthyléniques à chaîne latérale n-alkyle en C₁₂₋₅₀, de préférence en C₁₄₋₂₄ formant des homopolymères cristallins appelés dans la littérature anglo-saxonne *side chain cristalline polymers*. Il s'agit en particulier d'acrylates et de méthacrylates de n-alkyle en C₁₂₋₅₀ et de préférence en C₁₄₋₂₄.

Les motifs hydrophiles sont dérivés de préférence d'acides monocarboxyliques en C₃₋₆ α,β-insaturés tels que l'acide acrylique, l'acide méthacrylique ou l'acide crotonique, d'acides dicarboxyliques insaturés en C₄₋₆ tels que l'acide maléique et l'acide itaconique, ou des esters et amides à chaîne alkyle en C₁₋₄ de ces acides monocarboxyliques ou dicarboxyliques tels que les (méth)acrylates d'alkyle en C₁₋₄ et les N-(alkyle en C₁₋₄)-(meth)acrylamides.

La synthèse de ces polymères est décrite notamment dans la demande de brevet internationale WO 98/25710.

On peut également utiliser comme motif hydrophile le méthacrylate d'hydroxyéthyle ou la vinylpyrrolidone.

Les groupements acide carboxylique des motifs hydrophiles sont de préférence partiellement ou totalement neutralisés par une base choisie par exemple parmi la soude, la potasse, l'amino-2-méthyl-2-propanol, le monoéthanolamine, le triéthanolamine ou la triisopropanolamine.

Les polymères thermofusibles cristallins utilisables dans la présente invention sont par ailleurs caractérisés par un point de fusion cristalline relativement peu élevé, c'est-à-dire compris entre 30 et 80 °C.

Le point de fusion cristalline des polymères utilisables dans la présente invention est mesuré par analyse enthalpique différentielle (en anglais : *Differential Scanning Calorimetry*).

Cette méthode d'analyse thermique permet de mettre en évidence et de mesurer la cristallinité d'un polymère. L'enthalpie de fusion d'un polymère est la quantité d'énergie nécessaire pour transformer un échantillon partiellement ou totalement cristallin en un échantillon totalement amorphe. Le thermogramme ΔCp = f(T), dans lequel ΔCp représente la différence de capacité thermique de l'échantillon par rapport à un échantillon de référence ne subissant aucune transition thermique dans le domaine étudié, présente donc un signal endothermique dont l'aire est proportionnelle à l'enthalpie de fusion de l'échantillon. Seules les zones cristallines donnent lieu au phénomène de fusion.

Le point de fusion des polymères cristallins utilisables dans la présente invention est mesuré à l'aide d'un appareil d'analyse enthalpique différentielle (DSC), modèle M2920CE-5010 commercialisé par la société TA Instruments. On chauffe l'échantillon , à une vitesse de 10 °C/minute, de -20 °C à + 150 °C, et on enregistre la différence de capacité thermique entre l'échantillon et le témoin en fonction de la température.

On appelle point de fusion cristalline (T_{f}) la température correspondant au sommet du pic endothermique de fusion des zones cristallines ainsi obtenu.

On peut citer à titre d'exemple de polymères cristallins à bas point de fusion tels que ceux décrits ci-dessus, un copolymère statistique commercialisé sous la dénomination STRUCTURE® O par la société NATIONAL STARCH. Il s'agit d'un copolymère constitué de 10 % en poids de motifs dérivés d'acide acrylique et de 90 % en poids de motifs dérivés de méthacrylate de n-octadécyle. Ce copolymère a un point de fusion cristalline, mesuré par analyse enthalpique différentielle, égal à 46 °C.

Les compositions de coiffage de la présente invention contenant à la fois au moins un polymère filmogène fixant et au moins un polymère thermofusible cristallin sont des compositions liquides et peuvent être appliquées sur les cheveux par exemple à l'aide de moyens couramment utilisés dans le domaine du coiffage tels qu'un système aérosol ou un pulvérisateur.

La phase liquide de ces systèmes contient le ou les polymères fixants à l'état dissous ou dispersé et le ou les polymères thermofusibles cristallins à l'état dissous dans un solvant physiologiquement acceptable.

On peut citer à titre d'exemple de solvant physiologiquement acceptable l'eau, les alcools inférieurs en C₁₋₄ tels que l'éthanol ou l'isopropanol, l'acétone, la méthyléthylcétone, l'acétate d'éthyle, l'acétate de méthyle, l'acétate de butyle, le diéthoxyéthane, le diméthoxyéthane, les alcanes en C₆₋₁₀, ou des mélanges de ces composés.

La concentration totale en polymères de cette solution ou dispersion, c'est-à-dire la concentration en polymère filmogène fixant et en polymère thermofusible cristallin est de préférence comprise entre 1 et 20 % en poids rapportée à la composition de coiffage finale.

Les compositions coiffantes de la présente invention peuvent contenir en outre différents additifs ou adjuvants habituellement utilisés dans le domaine capillaire, tels que les épaississants, les agents tensioactifs anioniques, non ioniques, cationiques ou amphotères, les parfums, les conservateurs, les filtres solaires, les protéines, les vitamines, les provitamines, les polymères non fixants anioniques, non ioniques cationiques ou amphotères, les huiles minérales, végétales ou synthétiques, les céramides, les pseudocéramides, les silicones volatiles ou non volatiles, linéaires ou cycliques, modifiées ou non, et tout autre additif classiquement utilisé dans les compositions destinées à être appliquées sur les cheveux.

### Exemple

On prépare les trois compositions A, B et C suivantes :

| **Composition** | **A** | **B** | **C** |
|---|---|---|---|
| polymère cristallin à greffons aliphatiques "Structure O" commercialisé par la société National Starch | 4 % | - | 1 % |
| MEXOMERE PW (polymère fixant de la société CHIMEX) | - | 4 % | 3 % |
| 2-amino-2-méthyl-1-propanol | 0,5 % | 0,5 % | 0,5 % |
| éthanol absolu | 27 % | 27 % | 27 % |
| pentane | 33,5 % | 33,5 % | 33,5 % |
| diméthyléther | 35 % | 35% | 35 % |

On applique à l'aide d'un dispositif aérosol 3 g de chacune des compositions A, B et C ci-dessus sur une mèche de cheveux (2,5 g de cheveux européens châtains de 20 cm de long environ)

Après séchage à température ambiante, les mèches de cheveux sont démêlées à l'aide d'un peigne, puis chauffées sur un support plan pendant 5 secondes à l'aide d'un sèche cheveux placé à 10 cm. La température atteinte à la surface des cheveux lors du chauffage est de 80 °C environ. Les mèches sont ensuite laissées pendant 5 secondes à la température ambiante.

La mèche traitée avec la solution A contenant le polymère thermofusible cristallin seul conserve, certes, la forme plane imposée lors du chauffage mais sa cohésion entre les cheveux est totalement insatisfaisante en raison de l'extrême friabilité du polymère thermofusible cristallin.

La mèche traitée avec la solution B contenant uniquement le polymère fixant reste parfaitement floue et sans aucune cohésion.

La mèche traitée avec la solution C selon la présente invention contenant l'association d'un polymère fixant et d'un polymère thermofusible cristallin conserve la forme plane imposée lors de l'étape de chauffage et se distingue par une très bonne cohésion entre les fibres kératiniques. Cette mèche peut être remodelée (démêlage + chauffage au sèche cheveux + refroidissement) une dizaine de fois sans un nouvel apport de produit.

En conclusion, seul l'association du polymère fixant et du polymère thermofusible cristallin permet un remodelage de la mèche de cheveux.

## Revendications

1. Composition de coiffage contenant, dans un milieu liquide physiologiquement acceptable, l'association
(a) d'au moins un polymère filmogène fixant, et
(b) d'au moins un polymère thermofusible cristallin, soluble dans ledit milieu, ayant une température de fusion cristalline, mesurée par analyse enthalpique différentielle, comprise entre 30 °C et 80 °C, et constitué
i) de 85 à 98 % en poids de motifs hydrophobes dérivés de monomères α,β-éthyléniques à chaîne latérale n-alkyle en C₁₂₋₅₀, de préférence en C₁₄₋₂₄, formant des homopolymères cristallins et
ii) de 2 à 15 % en poids de motifs hydrophiles dérivés d'acides monocarboxyliques en C₃₋₆ α,β-insaturés, d'acides dicarboxyliques insaturés en C₄₋₆, des esters et amides à chaîne alkyle en C₁₋₄ de ces acides monocarboxyliques ou dicarboxyliques, du méthacrylate d'hydroxyéthyle et de la vinylpyrrolidone.

2. Composition de coiffage selon la revendication 1 ou 2, **caractérisée par le fait que** ledit polymère thermofusible cristallin est un copolymère contenant environ 10 % en poids de motifs dérivés d'acide acrylique et environ 90 % en poids de motifs dérivés de méthacrylate d'octadécyle.

3. Composition de coiffage selon l'une quelconque des revendications précédentes , **caractérisée par le fait que** ledit polymère filmogène fixant est choisi parmi les polymères filmogènes fixants anioniques, cationiques, amphotères ou non ioniques.

4. Composition de coiffage selon l'une quelconque des revendications précédentes, **caractérisée par le fait que** le rapport en poids dudit polymère thermofusible (b) au polymère fixant (a) est compris entre 1/10 000 et 3/1, de préférence entre 1/200 et 3/5.

5. Composition de coiffage selon l'une quelconque des revendications précédentes, **caractérisée par le fait que** la quantité totale de polymère fixant (a) et de polymère thermofusible (b) est comprise entre 1 % et 20 % en poids, rapportée à la composition de coiffage finale.

6. Composition de coiffage selon l'une quelconque des revendications précédentes, **caractérisée par le fait que** ledit milieu liquide physiologiquement acceptable comprend un solvant choisi parmi l'eau, les alcools inférieurs en C₁₋₄ tels que l'éthanol ou l'isopropanol, l'acétone, la méthyléthylcétone, l'acétate d'éthyle, l'acétate de méthyle, l'acétate de butyle, le diéthoxyéthane, le diméthoxyéthane, les alcanes en C₆₋₁₀, ou des mélanges de ces composés.

7. Procédé de remodelage des cheveux consistant
(1) à appliquer sur la chevelure sèche ou humide une composition de coiffage selon l'une quelconque des revendications précédentes,
(2) à donner à la chevelure la forme souhaitée en chauffant les cheveux au moyen d'une source de chaleur appropriée jusqu'à une température proche ou supérieure à la température de fusion cristalline du polymère thermofusible cristallin contenu dans la composition de coiffage, et
(3) à laisser refroidir la chevelure jusqu'à une température inférieure à ladite température de fusion cristalline dudit polymère thermofusible cristallin,
ces deux dernières étapes pouvant être répétées un grand nombre de fois sans apport de composition de coiffage supplémentaire.

## Patentansprüche

1. Zusammensetzung für die Frisurengestaltung, die in einem physiologisch akzeptablen.Medium die folgende Kombination enthält:
(a) mindestens ein fixierendes, filmbildendes Polymer und
(b) mindestens ein kristallines, in der Wärme schmelzbares Polymer, das in dem Medium löslich ist, eine mit dynamischer Differenz-Kalorimetrie ermittelte kristalline Schmelztemperatur im Bereich von 30 bis 80 °C aufweist und besteht aus:
(i) 85 bis 98 Gew.-% hydrophoben Einheiten, die von α,β-ethylenisch ungesättigten Monomeren mit n-Alkyl(C₁₂₋₅₀)-Seitenkette abgeleitet sind, wobei die Alkylgruppe vorzugsweise 14 bis 24 Kohlenstoffatome aufweist, welche kristalline Homopolymere bilden, und
(ii) 2 bis 15. Gew.-% hydrophile Einheiten, die von α,β-ungesättigten C₃₋₆-Monocarbonsäuren, ungesättigten C₄₋₆-Dicarbonsäuren, Estern und Amiden dieser Monocarbonsäuren oder Dicarbonsäuren mit C₁₋₄-Alkylkette, Hydroxyethylmethacrylat und Vinylpyrrolidon abgeleitet sind.

2. Zusammensetzung für die Frisurengestaltung nach Anspruch 1, **dadurch gekennzeichnet, dass** das kristalline, in der Wärme schmelzbare Polymer ein Copolymer ist, das etwa 10 Gew.-% von Acrylsäure abgeleitete Einheiten und etwa 90 Gew.-% von Octadecylmethacrylat abgeleitete Einheiten enthält.

3. Zusammensetzung für die Frisurengestaltung nach einem der vorhergehenden Ansprüche, **dadurch gekennzeichnet, dass** das festigende film bildende Polymer unter den anionischen, kationischen, amphoteren oder nichtionischen fixierenden filmbildenden Polymeren ausgewählt ist.

4. Zusammensetzung für die Frisurengestaltung nach einem der vorhergehenden Ansprüche, **dadurch gekennzeichnet, dass** das Gewichtsverhältnis des in der Wärme schmelzbaren Polymers (b) und des fixierenden Polymers (a) im Bereich von 1/10.000 bis 3/1 und vorzugsweise 1/200 bis 3/5 liegt.

5. Zusammensetzung für die Frisurengestaltung nach einem der vorhergehenden Ansprüche, **dadurch gekennzeichnet, dass** die Gesamtmenge des festigenden Polymers (a) und des in der Wärme schmelzbaren Polymers (b) im Bereich von 1 bis 20 Gew.-%, bezogen auf die fertige Zusammensetzung für die Frisurengestaltung, liegt.

6. Zusammensetzung für die Frisurengestaltung nach einem der vorhergehenden Ansprüche, **dadurch gekennzeichnet, dass** das physiologisch akzeptable flüssige Medium ein Lösungsmittel enthält, das unter Wasser, niederen C₁₋₄-Alkoholen, wie Ethanol oder Isopropanol, Aceton, Methylethylketon, Ethylacetat, Methylacetat, Butylacetat, Diethoxyethan, Dimethoxyethan, C₆₋₁₀-Alkanen oder den Gemischen dieser Verbindungen ausgewählt ist.

7. Verfahren zur wiederholten Formung der Haare, das darin besteht:
(1) auf die trockenen oder feuchten Haare eine Zusammensetzung für die Frisurengestaltung nach einem der vorhergehenden Ansprüche aufzubringen,
(2) den Haaren die gewünschte Form zu geben, indem die Haare mit einer geeigneten Wärmequelle bis auf eine Temperatur in der Nähe der kristallinen Schmelztemperatur des in der Wärme schmelzbaren, kristallinen Polymers, das in der Zusammensetzung zur Frisurengestaltung enthalten ist, oder über diese Temperatur erwärmt werden, und
(3) die Haare auf eine Temperatur abkühlen zu lassen, die unter der kristallinen Schmelztemperatur des in der Wärme schmelzbaren, kristallinen Polymers liegt,
wobei die beiden letzten Schritte mehrmals wiederholt werden können, ohne dass die Zusammensetzung zur Frisurengestaltung erneut aufgebracht werden muss.

## Claims

1. Styling composition comprising, in a physiologically acceptable medium, the combination
(a) of at least one film-forming fixing polymer and
(b) of at least one crystalline hot-melt polymer which is soluble in the said medium, which has a crystalline melting temperature, measured by differential scanning calorimetry, of between 30°C and 80°C, and which is composed
i) of 85 to 98% by weight of hydrophobic units derived from α,β-ethylenic monomers with a C₁₂₋₅₀, preferably C₁₄₋₂₄, n-alkyl side chain which form crystalline homopolymers and
ii) of 2 to 15% by weight of hydrophilic units derived from α,β-unsaturated C₃₋₆ monocarboxylic acids, from unsaturated C₄₋₆ dicarboxylic acids, from the esters and amides comprising a C₁₋₄ alkyl chain of these monocarboxylic or dicarboxylic acids, from hydroxyethyl methacrylate and from vinylpyrrolidone.

2. Styling composition according to Claim 1, **characterized in that** the said crystalline hot-melt polymer is a copolymer comprising approximately 10% by weight of units derived from acrylic acid and approximately 90% by weight of units derived from octadecyl methacrylate.

3. Styling composition according to either one of the preceding claims, **characterized in that** the said film-forming fixing polymer is chosen from anionic, cationic, amphoteric or nonionic film-forming fixing polymers.

4. Styling composition according to any one of the preceding claims, **characterized in that** the ratio by weight of the said hot-melt polymer (b) to the fixing polymer (a) is between 1/10 000 and 3/1, preferably between 1/200 and 3/5.

5. Styling composition according to any one of the preceding claims, **characterized in that** the total amount of fixing polymer (a) and of hot-melt polymer (b) is between 1% and 20% by weight with respect to the final styling composition.

6. Styling composition according to any one of the preceding claims, **characterized in that** the said physiologically acceptable liquid medium comprises a solvent chosen from water, lower C₁₋₄ alcohols, such as ethanol or isopropanol, acetone, methyl ethyl ketone, ethyl acetate, methyl acetate, butyl acetate, diethoxyethane, dimethoxyethane, C₆₋₁₀ alkanes or mixtures of these compounds.

7. Process for remodelling the hair, which consists
(1) in applying, to dry or wet hair, a styling composition according to any one of the preceding claims,
(2) in giving the hair the desired shape by heating the hair by means of an appropriate heat source to a temperature close to or greater than the crystalline melting temperature of the crystalline hot-melt polymer present in the styling composition, and
(3) in allowing the hair to cool to a temperature below the said crystalline melting temperature of the said crystalline hot-melt polymer, it being possible for these latter two stages to be repeated many times without the introduction of additional styling composition.
